(19) Europäisches Patentamt
European Patent Office
Office européen des brevets

(11) EP 1 437 145 A1

# (12) EUROPEAN PATENT APPLICATION

(43) Date of publication:
**14.07.2004 Bulletin 2004/29**

(51) Int Cl.[7]: **A61K 51/08**

(21) Application number: **03000204.2**

(22) Date of filing: **07.01.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicant: **Schering AG**
**13353 Berlin-Wedding (DE)**

(72) Inventors:
- **Manchanda, Rajesh**
  **Brookline, MA 02445-7116 (US)**
- **Krause, Sabine**
  **13156 Berlin (DE)**

(74) Representative: **Dörries, Hans Ulrich, Dr. et al**
**Dörries, Frank-Molnia & Pohlman,**
**Triftstrasse 13**
**80538 München (DE)**

## (54) Enhanced scintigraphic imaging agents for imaging of infection and inflammation

(57) The invention describes enhanced scintigraphic imaging agents that can be used to localize infection and inflammation in a mammal. Specifically, the invention relates to radiolabeled, preferably technetium-99m labeled scintigraphic imaging agents that are compositions of a polysulfated glycan or mixture thereof and a compound comprising a polybasic peptide covalently linked to a radiolabel binding moiety. Methods and kits for making such compositions, and methods for using such compositions to image sites of infection and inflammation in a mammalian body are also provided. The enhanced imaging agents exhibit improved binding affinity to the polysulfated glycans, better biodistribution and infection uptake, thus providing improved imaging results.

EP 1 437 145 A1

## Description

This invention relates to reagents, compositions, and scintigraphic imaging agents useful for example to localize infection or inflammation, particularly in a mammalian body. Specifically, this invention relates to reagents, compositions, and enhanced imaging agents that comprise a polybasic peptide having at least four arginine residues and a radiolabel-binding moiety covalently linked to the peptide, the compositions further comprising a polysulfated glycan such as dermatan sulfate and dermatan disulfate, optionally radiolabeled with a radioisotope such as technetium-99m (Tc-99m). The enhanced imaging agents exhibit increased binding affinity to the polysulfated glycans and better biodistribution with improved infection uptake, thus leading to better imaging results. Also included in the invention are methods and kits for making such agents and compositions and methods of using said reagents, compositions and imaging agents to image sites of infection and inflammation, particularly in the mammalian body.

## Background of the Invention

There is a clinical need to be able to determine the location and/or extent of sites of focal or localized infection and inflammation. In a substantial number of cases, conventional methods of diagnosis (X-ray, physical examination, CT and ultrasonography) fail to identify certain sites, such as an abscess. Although biopsy may be resorted to, it is preferable to avoid such invasive procedures, at least until they are diagnostically appropriate to identify the pathogen responsible for an abscess at a known location. Identification of the site of the infection is important because rapid localization and identification of the problem is critical to effective therapeutic intervention.

In the field of nuclear medicine, certain pathological conditions can be localized or the extent of such conditions determined by imaging the internal distribution of administered radioactively-labeled tracer compounds (i.e. radiotracers or radiopharmaceuticals) that accumulate specifically at the pathological site. A variety of radionuclides are known to be useful for radioimaging, including $^{67}$Ga, $^{99m}$Tc (Tc-99m), $^{111}$In, $^{123}$I, $^{125}$I, $^{169}$Yb and $^{186}$Re.

However, an abscess may be caused by any one of many possible pathogens, so that a radiotracer specific for a particular pathogen would have limited scope. On the other hand, infection is almost invariably accompanied by inflammation, which is a general response of the body to tissue injury. Therefore, a radiotracer specific for sites of inflammation would be expected to be useful in localizing sites of infection caused by any pathogen, as well as being useful for localizing other inflammatory sites.

One of the main phenomena associated with inflammation is the localization of leukocytes, usually monocytes and neutrophils, at the site of inflammation. Radiotracers specific for leukocytes would be useful in detecting leukocytes at the sites of localized infection. However, the direct radiolabeling of leukocytes, e.g. with $^{111}$In, involves a number of technical steps and a delay of 12 to 48 hours between injection and imaging to obtain optimal results. While Tc-99m labeled leukocytes have been used to shorten this delay period (see, e.g. Vorne et al., 1989, J. Nucl. Med. 30: 1332-1336), extra-corporeal labeling is still required. A preferred radiotracer would be one that either would label leukocytes in whole blood or would not require removal and manipulation of autologous blood components ex corpora.

One alternative approach to the radiolabeling of leukocytes is the use of radiolabeled peptides that specifically bind to leukocytes with high affinity. This approach avoids the problems inherent in the removal and labeling of leukocytes. One class of peptides known to bind to leukocytes are chemotactic peptides. These peptides bind to receptors on the surface of leukocytes with high affinity. One particular peptide which has been extensively studied and shown to bind to leukocytes with high affinity is Platelet Factor 4.

Platelet Factor 4 (PF4) is a naturally-occurring chemotactic peptide consisting of 70 amino acids and is known in the prior art to be chemotactic and to bind to neutrophils and monocytes (Deuel et al., 1981, *Proc. Natl. Acad. Sci.,* 78:4584-4587), cell types known to be associated with sites of infection and inflammation *in vivo.* PF4 is a 7,8 kDa polypeptide that is released from platelets upon degranulation and aids in neutralizing heparin. The amino acid sequence of PF4 has been determined (Deuel et al., 1977, *Proc. Natl. Acad. Sci.,* 74:2256-2258). The C-terminus of PF4 binds to heparin with high affinity. (Loscalzo et al., 1985, *Arch. Biochem. Biophys.,* 246:446-455). Moreover, the C-terminus of PF4 possesses higher monocyte chemotactic potency (Osterman et al., 1982, *Biochem. Biophys. Res. Comm.* 107: 130-135). Holt & Niewiarowski, 1985, Sem. Hematol. 22: 151-163 provide a review of the biochemistry of platelet a-granule proteins, including platelet factor 4 and Goldman et al., 1985, Immunol. 54: 163-171 reveal that fMLF receptor-mediated uptake is inhibited in human neutrophils by platelet factor 4 and a carboxy-terminal dodecapeptide thereof.

Thorbecke & Zucker, 1989, EP Publ. No. EP-A-0 301 458, disclose compositions and methods for modulating immune responses comprising administering an immunomodulating amount of platelet factor 4 or peptides derived therefrom.

The use of chelating agents for radiolabeling polypeptides, and methods for labeling peptides and polypeptides with Tc-99m are known in the prior art and are disclosed in U.S. Pat. No. 5,654,272; U.S. Pat. No. 5,443,815; U. S. Pat. No. 5,720,934; U.S. Pat. No. 5,508,020; U.S. Pat. No. 5,552,525; U.S. Pat. No. 5,645,815; U.S. Pat. No.

5,561,220; and PCT International Applications PCT/US92/10716, PCT/US93/02320 and PCT/US93/04794, which are hereby incorporated by reference.

P483 is a 23-amino acid peptide derivative of the heparin-binding tridecapeptide C-terminus of PF4. The amino acid sequence of P483 is shown below with the PF4 mimic sequence in italics:

Acetyl-LysLysLysLysLys**CysGlyCys**GlyGly*ProLeuTyrLysLysIleIleLysLysLeuLeuGluSer*

(SEQ ID No. 1).

P483 contains a CGC sequence (depicted in bold letters) for binding of Tc-99m and contains an acetylated N-terminus comprised of five lysine residues. Similar to PF4, P483 also binds to heparin to form a peptide-heparin complex (PHC), P483H. Compositions comprising P483H complexes and the use of Tc-99m-labeled compositions (Tc-99m P483H) in imaging applications have been disclosed in U.S. 6,019,958.

However, there continues to be a need for scintigraphic imaging agents that can provide better imaging results such as a higher image contrast.

## Summary of the Invention

The present invention provides enhanced scintigraphic imaging agents that are compositions comprising radioactively-labeled reagents and polysulfated glycans. The compositions of the invention accumulate at sites of a pathology such as inflammation *in vivo.* The reagents comprised in the compositions of the invention and useful for their preparation, are themselves comprised of a polybasic compound comprising a peptide that are capable, preferably by virtue of the peptide, of specifically localizing at sites of infection or inflammation, wherein said peptides are covalently linked to radiolabel binding, preferably technetium-99m-binding, moieties.

The combination of a radiolabeled polybasic compound comprising a peptide and a polysulfated glycan as provided by the present invention advantageously enables the acquisition of high quality scintigraphic images of focal sites of infection and inflammation *in vivo.* Administration of this combination results in a greater degree of localization of the radioactive signal of the radioisotope such as Tc-99m at the site of infection when compared to administration of prior art agents or the radiolabeled polybasic compound alone.

It has now surprisingly been found that polybasic peptide compounds according to the present invention, e. g. fragments of PF4, or P483, having arginine residues in addition or instead of lysine residues exhibit an increased binding affinity to heparin and other polysulfated glycans, thus yielding enhanced binding of the peptide to the polysulfated glycan, and that coadministration thereof leads to more favorable biodistribution and increased image contrast values. Moreover, the exchange of heparin with a polysulfated glycan comprising a higher and more homogeneous sulfation yields even more favorable biodistribution and higher image contrast values.

Accordingly, the invention provides radiolabeled and unlabeled compositions that accumulate at sites of inflammation *in vivo,* reagents and methods for preparing said compositions, and methods for using said radiolabeled compositions for imaging sites of infection and inflammation within a mammalian body.

In a first aspect of the present invention, reagents are provided that comprise (a) a polybasic compound comprising a peptide, wherein the peptide comprises at least four arginine residues; and (b) a radiolabel-binding moiety covalently linked to the polybasic compound, wherein the reagent is capable of accumulating at sites of pathology in the body. Preferred are reagents wherein the polybasic compound is said peptide comprising at least four arginine residues. The compositions according to the present invention are capable of accumulating at sites of pathology in the body, preferably by virtue of the peptides comprised therein. In particularly preferred embodiments, the compositions are capable of accumulating at sites of inflammation or infection, i.e., said compositions may bind to leukocytes, preferably monocytes and neutrophils and most preferably to neutrophils *in vivo.*

For the purposes of this invention, the term "accumulation at sites of infection or inflammation *in vivo"* is intended to mean that the compositions of the invention are capable of accumulating at sites of infection or inflammation in the mammalian body in such a way so as to allow detection of accumulated radiolabeled complexes prepared from the compositions as disclosed herein at sites of infection or inflammation by gamma scintigraphy.

Each polybasic peptide-containing embodiment of the invention comprises a sequence of amino acids. The term amino acid as used in this invention is intended to include all L- and D-amino acids, naturally occurring and otherwise. Preferred are embodiments, wherein the amino acids are naturally occurring L-amino acids.

Preferably, the peptide has from about 5 to about 100 amino acids. In a preferred embodiment of the present invention, said reagents comprise a peptide, wherein the peptide comprises an amino acid sequence corresponding to a sequence of about 5 to 70, preferably about 50, 40, 30, 20, 15, 14, 13, 12, 11, 10, or 9 contiguous amino acids of

human Platelet Factor 4, or having at least 40, 50, 60, 70, 80, 90, 95, 96, 97, 98, or 99% sequence identity to said sequence. The sequence of contiguous amino acids is preferably from the C-terminus of human Platelet Factor 4 (PF4).

Peptides useful in the practice of this invention include those capable of accumulating at sites of infection and inflammation in a mammalian body. Examples of such peptides and reagents are presented hereinafter in the Examples.

The enhanced scintigraphic imaging agents comprising the polybasic compounds of the present invention yield enhanced imaging as a result of the presence of arginine residues, in particular when administered in a composition further comprising a polysulfated glycan, as explained in more detail below.

In embodiments where the peptide comprises a PF4 sequence or a fragment or analog thereof, the at least four arginine residues of the polybasic compound may either represent a substitution of corresponding lysine residues in the amino acid sequence of human PF4, or represent an addition to the amino acid sequence corresponding to said sequence of human PF4. Preferably, the arginine residues represent substitutions of lysine residues, but any combination of addition and substitution is also within the scope of the present invention.

In principle, the polybasic compounds of the present invention may comprise any number of arginine residues. However, it is preferred that at least four, and preferably five, or six, more preferably seven, or eight, and most preferably nine arginine residues are present in the reagents of the present invention.

In a second aspect, the present invention provides reagents comprising a polybasic compound covalently linked to a radiolabel-binding moiety of formula

I.

Cp(aa)Cp

wherein Cp is a protected or unprotected cysteine residue and (aa) stands for any amino acid. In a preferred embodiment, the amino acid is an alpha amino acid and most preferably is glycine.

When Cp represents a protected cysteine, the S-protecting groups are the same or different and may be but are not limited to:

- $-CH_2$-aryl (aryl is phenyl or alkyl or alkyloxy substituted phenyl);
- $-CH-(aryl)_2$, (aryl is phenyl or alkyl or alkyloxy substituted phenyl);
- $-C-(aryl)_3$, (aryl is phenyl or alkyl or alkyloxy substituted phenyl);
- $CH_2$-(4-methoxyphenyl);
- CH-(4-pyridyl)$(phenyl)_2$;
- $C(CH_3)_3$
- 9-phenylfluorenyl;
- $CH_2$-NHCOR (R is unsubstituted or substituted alkyl or aryl);
- $CH_2$-NHCOR (R is a lower alkyl having 1 to 6 carbon atoms, 2-pyridyl, 3-pyridyl, 4-pyridyl, phenyl, or phenyl substitute with lower alkyl, hydroxy, lower alkoxy, carboxy, or lower alkoxycarbonyl);
- $CH_2$-NHCOOR (R is unsubstituted or substituted alkyl or aryl);
- CONHR (R is unsubstituted or substituted alkyl or aryl);
- $CH_2$-S-$CH_2$-phenyl.

Radiolabel-binding moieties comprising cysteine-sulfur protecting groups designated "$(pgp)^s$", such as the bisamino, bisthiol moieties of the invention, are also described by the above-mentioned listing of protecting groups.

A preferred protecting group has the formula $-CH_2$-NHCOR wherein R is a lower alkyl, 2-pyridyl, 3-pyridyl, 4-pyridyl, phenyl, or phenyl substitute with lower alkyl, hydroxy, lower alkoxy, carboxy, or lower alkoxycarbonyl.

When using the term "lower" alkyl, alkoxy, carboxy, or alkoxycarbonyl and the like, it should be understood that "lower" is meant throughout the specification and the claims as having from 1 to 6 carbon atoms in the respective residue. In a preferred embodiment, lower alkyl is a $CH_3$ or $C_2H_5$ group. Accordingly, the most preferred protecting group is an acetamidomethyl group.

Another preferred type of radiolabel-binding moiety of the reagents of the present invention is a single thiol-group containing moiety having the following formula:

II.

$$A\text{-}CZ(B)\text{-}[C(R^1R^2)]_{n}\text{-}X$$

wherein A is H, HOOC, $H_2NOC$, (peptide)-NHOC, (peptide)-OOC or $R^4$; B is H, SH, $-NHR^3$, $-N(R^3)$-(peptide), or $R^4$; X is H, SH, $-NHR^3$, $-N(R^3)$-(peptide) or $R^4$;

Z is H or $R^4$; $R^1$, $R^2$, $R^3$ and $R^4$ are independently H or lower straight or branched chain or cyclic alkyl; n is 0, 1 or 2; and

(1) where B is $-NHR^3$ or $-N(R^3)$-(peptide), X is SH, and n is 1 or 2;

(2) where X is $-NHR^3$ or $-N(R^3)$-(peptide), B is SH, and n is 1 or 2;

(3) where B is H or $R^4$, A is HOOC, $H_2NOC$, (peptide)-NHOC, or (peptide)-OOC, X is SH, and n is 0 or 1;

(4) where A is H or $R^4$, then where B is SH, X is $-NHR^3$ or $-N(R^3)$-(peptide);

(5) where X is SH, B is $-NHR^3$ or $-N(R^3)$-(peptide);

(6) where X is H or $R^4$, A is HOOC, $H_2NOC$, (peptide)-NHOC, or (peptide)-OOC and B is SH;

(7) where Z is methyl, X is methyl, A is HOOC, $H_2NOC$, (peptide)-NHOC, or (peptide)-OOC, B is SH and n is 0;

and wherein the thiol moiety is in the reduced form;

Yet another preferred radiolabel-binding moiety of the reagents of the present invention has one of the following formulae:

III.

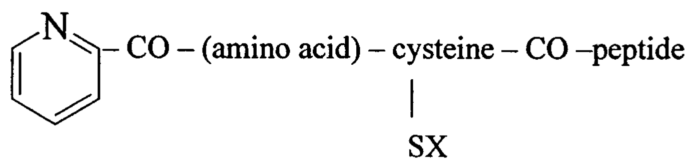

or

IV.

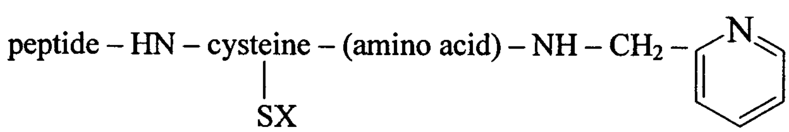

wherein X is H or a protecting group and (amino acid) represents any amino acid; or

V.

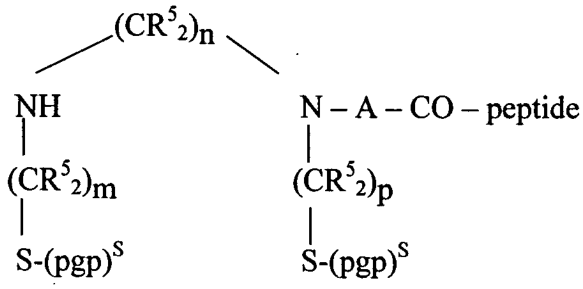

wherein each $R^5$ is independently H, lower alkyl, phenyl, or phenyl substituted with lower alkyl or lower alkoxy, and each $(pgp)^s$ is independently a thiol protecting group or H; m, n and p are independently 2 or 3, and A represents linear or cyclic lower alkyl, aryl, heterocyclyl, combinations or substituted derivatives thereof; or

VI.

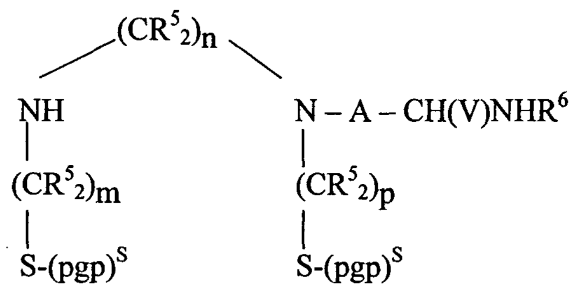

wherein each $R^5$ is independently H, lower alkyl, phenyl, or phenyl substituted with lower alkyl or lower alkoxy and each $(pgp)^s$ is independently a thiol protecting group or H; m, n and p are independently 1, 2 or 3, and A is linear or cyclic lower alkyl, aryl, heterocyclyl, or combinations or substituted derivatives thereof, V is H or -CO-peptide, $R^6$ is H or a peptide; and wherein when V is H, then $R^6$ is a peptide and when $R^6$ is H, then V is -CO-peptide.

In certain preferred embodiments of the invention, the above-mentioned radiolabel-binding moieties may be covalently linked to the polybasic compound through from about one to about twenty amino acids. A particularly preferred amino acid for covalently linking the polybasic compound and the radiolabel-binding moiety is glycine, but in general, any amino acid may be used.

In yet another preferred embodiment, the reagent comprises the amino acid sequence of P483, i.e.,

KKKKKCGCGGPLYKKIIKKLLES (SEQ ID No. 2)

except that at least four, preferably five, six, seven, eight and most preferably nine of the lysine residues of said peptide are substituted by arginine residues.

Particularly suitable embodiments of the present invention comprise reagents wherein the polybasic compound and the radiolabel-binding moiety covalently linked thereto together form a peptide having an amino acid sequence selected from the group consisting of:

Acetyl-RRRRRCGCGGPLYRRIIRRLLES (SEQ ID No. 3);

Acetyl-RRRRRCGCGGPLYKKIIKKLLES (SEQ ID No. 4);

and

Acetyl-KKKKKCGCGGPLYRRIIRRLLES (SEQ ID No. 5).

In another aspect of the invention, the reagents of the invention may also comprise a polyvalent linking moiety, thus resulting in a multimeric reagent that comprises (a) at least two polybasic compounds as described above, which may be identical or different, (b) at least one radiolabel-binding moiety as described above covalently linked to at least one of the polybasic compounds, and (c) a polyvalent linker moiety covalently linked to the polybasic compounds, the radiolabel-binding moieties or both, wherein the molecular weight of the multimeric polyvalent reagent is less than about 20,000 Da.

Among the preferred embodiments of this aspect of the invention are those polyvalent linking moieties that are comprised of at least 2 linker functional groups capable of covalently bonding to the polybasic compounds or the radiolabel-binding moieties, preferably wherein at least 2 of the linker functional groups are identical. Optionally, the linker functional groups may be primary or secondary amines, hydroxyl groups, carboxylic acid groups or thiol-reactive groups, the thiol-reactive groups being selected from maleimido groups and chloroacetyl, bromoacetyl and iodoacetyl groups.

Most preferred in this aspect of the invention are multimeric reagents, wherein the polyvalent linker is selected from *bis*-succinimidylmethylether (BSME), 4-(2,2-dimethylacetyl)benzoic acid (DMBA), *tris*(succinimidylethyl)amine (TSEA), *bis*-succinimidohexane (BSH), 4-(O-$H_2$CO-Gly-Gly-Cys.amide)acetophenone (ETAC), *tris*(acetamidoethyl) amine (TAEA), *bis*(acetamidomethyl)amine, *bis*(acetamidoethyl)amine, $\alpha,\varepsilon$-*bis*(acetyl)lysine, lysine, 1,8-*bis*-acetamido-3,6-dioxaoctane, or a derivative of any of the above-listed polyvalent linkers.

The above-described reagents are useful components for preparing the enhanced imaging agents of the present invention.

In yet another aspect of the present invention, compositions are provided that comprise (a) a reagent as described above, including any of the reagents that may represent one of the preferred embodiments described above, and (b) a polysulfated glycan having a molecular weight of at least about 1000 Da, wherein the composition is capable of accumulating at sites of pathology such as infection and/or inflammation in a mammalian body.

Particularly suitable polysulfated glycans in this aspect of the invention comprise dextran sulfate, chondroitin sulfate, dermatan sulfate, dermatan disulfate, or any derivatives or mixtures thereof, although other known polysulfated glycans such as heparin or heparan sulfate may also be used. Particularly preferred polysulfated glycans in the compositions of the present invention are those polysulfated glycans having a constant carboxyl:sulfate ratio, such as dermatan sulfate or dermatan disulfate.

Heparin is a linear chain polymer of 2-deoxy-2-aminoglucopyranose and hexuronic acid. Heparin Sodium USP is isolated from porcine intestinal mucosa and exhibits a molecular weight distribution ranging from 5 to 40 kDa with varying levels of sulfation. The negatively-charged sulfate groups of heparin are reported to bind to the positively-charged lysine residues on PF4 (and P483).

In contrast to heparin, dermatan sulfate (DS) is a linear homogeneous chain polymer consisting of repeating units of the same disaccharide moiety consisting of beta-iduronic acid and N-acetyl-galactosamine-4-sulfate. Dermatan sulfate is purified as a larger molecular weight fraction during the purification of heparin from porcine intestinal mucosa. The level of sulfation in DS is consistent because it contains only one sulfate group per disaccharide unit (carboxyl to sulfate ratio = 1.4). Dermatan Disulfate (DDS) is a site-specific hypersulfated derivative of DS. In DDS, the 6-position of N-acetyl-$\beta$-beta-galactosamine-4-sulfate is also sulfated. The carboxyl to sulfate ratio in DDS is 1.7 and DDS retains the structural/physiochemical homogeneity of DS. Both DS and DDS bind with high affinity to P483 and to the polybasic peptides of the invention forming so-called protein glycan complexes (PGC's).

The determination of a suitable (weight/weight) ratio of the polybasic compound to the polysulfated glycan is within the skill of those skilled in the art. Particularly preferred is a range from 0.1:1 to 20:1, preferably from 0.2:1 to 10:1, 0.5 to 5, or 1:1 to 2:1, and is most preferably about or exactly 1.45:1 or 1.5:1.

The compositions of the present invention accumulate at sites of inflammation in *vivo* and are thus useful for acquiring scintigraphic images when labeled with a suitable radioisotope, such as Tc-99m.

In yet another aspect of the present invention, the compositions of the invention are capable of achieving a favorable biodistribution that is characterized by a specific accumulation at the site to be imaged, e.g. in an infected muscle, and at the same time a low concentration in uninfected areas, such as normal muscle or blood. The biodistribution of the radiolabeled compositions of the invention may be expressed by means of an image contrast ratio. The image contrast ratio can be determined as the ratio $I_{max}$(infected muscle) / $I_{max}$(control muscle), hereinafter referred to as $I_{max}$:C, i.e., the maximum radioactivity accumulated in an infected muscle sample versus the maximum radioactivity accumulated in a control, i.e., uninfected, muscle sample. The radioactivity is measured as % of injected dose per gram of tissue/blood, i.e., %ID/g. For the determination of $I_{max}$ values (and $I_{avg}$ values; see below), the tissue or blood samples are divided into six parts of equal size / weight and the radioactivity in each of the six samples is counted. The sample with the highest radioactivity is used for the $I_{max}$ value (the average of all six samples was used for $I_{avg}$). $I_{max}$ (and $I_{avg}$) values are expressed as %ID/g.

Alternatively, the image contrast ratio can be determined as the ratio $I_{max}$(infected muscle) / $I_{max}$(blood), hereinafter referred to as $I_{max}$:B, i.e., the maximum radioactivity accumulated in infected muscle versus the maximum radioactivity accumulated in the blood. Again, the radioactivity is measured as % of injected dose per gram of tissue/ blood, i.e., %ID/g. The determination of the image contrast ratios and results for certain embodiments of the present invention are described in the Examples.

Preferred compositions of the invention are capable of achieving an image contrast ratio $I_{max}$:C between muscle tissue infected by *E. coli* and uninfected muscle tissue in the rabbit injection model described herein (see Examples 5 to 8) of more than 25, preferably more than 40, and most preferably more than 60, when the reagent of the composition is labeled with Tc-99m and administered together with the polysulfated glycan.

Alternatively, the preferred compositions of the invention are capable of achieving an image contrast ratio $I_{max}$:B between muscle tissue infected by *E. coli* and terminal blood in the rabbit injection model described herein (see Examples 5 to 8) of more than 3, preferably more than 4, 5, 6, 7, or 8 and most preferably more than 9, when the reagent of the composition is labeled with Tc-99m and administered together with the polysulfated glycan.

In yet another aspect of the present invention, the present invention also provides scintigraphic imaging agents that comprise any of the above-described compositions of the present invention and a radioisotope complexed to the reagent of the composition via its radiolabel-binding moiety. The imaging agents of the invention specifically bind to sites of pathology, e.g., inflammation or infection *in vivo.* The combination of radiolabeled polybasic peptide-containing compounds and a polysulfated glycan, such as dermatan sulfate, enables the acquisition of improved scintigraphic images at sites of infection and inflammation.

The radioactive label is preferably technetium-99m, although other radioisotopes such as fluor-18, gallium-67, gallium-68, indium-111, iodine-123, iodine-125, ytterbium-169, or rhenium-186 may also be used to label the reagents of the invention.

The possibility of labeling with Tc-99m is an advantage of the present invention because the nuclear and radioactive properties of this isotope make it an ideal component of a scintigraphic imaging agent. The isotope has a single photon energy of 140 keV and a radioactive half-life of about 6 hours, and is readily available from a Mo-$^{99m}$Tc generator (Pinkerton et al., 1985, Journal of Chemical Education, 62:965-973). Other radionuclides known in the prior art have effective half-lives which are much longer or may be toxic.

Administration of these radiolabeled, in particular Tc-99m labeled reagents in combination with a polysulfated glycan, such as dermatan sulfate, results in a greater degree of localization of the radioactive signal at the site of infection, better biodistribution and image contrast. Thus, the scintigraphic images produced are superior to the images obtained using the peptides known from the prior art.

In another aspect, pharmaceutical compositions comprising the above reagents, compositions or radiolabeled scintigraphic imaging agents and one ore more pharmaceutically acceptable carriers are also provided by the present invention. The reagents, compositions, radiolabeled scintigraphic imaging agents, or pharmaceutical compositions may also be used for preparing a diagnostic pharmaceutical suitable for imaging a site of pathology, such as inflammation or infection, within the mammalian body.

The present invention also provides methods for preparing the reagents of the scintigraphic imaging agents by *in vitro* chemical synthesis. The peptide embodiments of the reagents of the invention can be chemically synthesized using methods and means well-known to those with skill in the art and described herein below. In a preferred embodiment, peptides are synthesized by solid phase peptide synthesis. (Fields et al., Principles and Practice of Solid-Phase-Peptide Synthesis, in *Synthetic peptides,* 2002, Oxford University Press).

Radiolabel-binding moieties of the invention may be covalently linked to the target specific polybasic compound comprising a peptide during peptide synthesis. For embodiments [e.g., Pic-Gly-Cys(protecting group)-] comprising picolinic acid (Pic-), the radiolabel-binding moiety can be synthesized as the last (i.e., amino-terminal) residue in the synthesis. In addition, the picolinic acid-containing radiolabel-binding moiety may be covalently linked to the epsilon-amino group of lysine to give, for example, αN(Fmoc)-Lys-εN[Pic-Gly-Cys(protecting group)], which may be incorporated at any position in the peptide chain. This sequence is particularly advantageous as it affords an easy

mode of incorporation into the target binding peptide.

Similarly, the picolylamine (Pica)-containing radiolabel-binding moiety [-Cys(protecting group)-Gly-Pica] can be prepared during peptide synthesis by including the sequence [-Cys(protecting group)-Gly-] at the carboxyl terminus of the peptide chain. Following cleavage of the peptide from the resin the carboxy terminus of the peptide is activated and coupled to picolylamine. This synthetic route requires that reactive side-chain functionalities remain masked (protected) and do not react during the conjugation of the picolylamine.

This invention provides for the incorporation of these radiolabel-binding moieties (chelators) into virtually any polybasic peptide, resulting in radiolabeled peptide components of the scintigraphic imaging agent compositions of the invention.

Radiolabeled complexes provided by the invention are formed by reacting the reagents of the invention with the radionuclide, such as Tc-99m, the latter preferably in form of a salt of Tc-99m pertechnetate, in the presence of a reducing agent. Preferred reducing agents include but are not limited to dithionite ions, stannous ions and ferrous ions, or may be a solid-phase reducing agent. Alternatively, the complex may be formed by reacting a reagent of the invention with a pre-formed labile complex of technetium and another compound known as a transfer ligand. This process is known as ligand exchange and is well known to those skilled in the art. The labile complex may be formed using such transfer ligands as tartrate, citrate, gluconate or mannitol, for example. Among the Tc-99m pertechnetate salts useful with the present invention are included the alkali metal salts such as the sodium salt, or ammonium salts or lower alkyl ammonium salts. The reaction of the reagents of the invention with Tc-99m pertechnetate or preformed Tc-99m labile complex can be carried out in an aqueous medium at room temperature. When an anionic complex is formed in an aqueous medium, the radiolabeled complex is in the form of a salt with a suitable cation such as sodium cation, ammonium cation, mono, di- or tri-lower alkyl amine cation, or any pharmaceutically acceptable cation. Complexes with other radiolabels can be similarly prepared.

Radioactively labeled scintigraphic imaging agents provided by the present invention are provided having a suitable amount of radioactivity. In forming the radioactive complexes, it is generally preferred to form radioactive complexes in solutions comprising radioactivity at concentrations of from about 0.01 millicurie (mCi) to 100 mCi per ml.

Compositions comprising scintigraphic imaging agents comprised of radiolabeled reagents and polysulfated glycans provided by this invention can be used for visualizing sites of inflammation, including abscesses and sites of "occult" infection. The radiolabeled compositions can also be used for visualizing sites of inflammation caused by tissue ischemia, including such disorders as inflammatory bowel diseases and arthritis.

The radiolabeled pharmaceutical compositions of the invention may be administered intravenously, in any conventional medium for intravenous injection such as an aqueous saline medium, or in blood plasma medium to diagnostically image various organs, pathogenicities and the like in accordance with this invention. Such medium may also contain conventional pharmaceutical adjunct materials such as, for example, pharmaceutically acceptable salts to adjust the osmotic pressure, buffers, preservatives and the like. Among the preferred media are normal saline and plasma.

In accordance with this invention, the radiolabeled compositions, wherein the reagents are provided either as a complex or as a salt with a pharmaceutically acceptable counterion, are administered in a single unit injectable dose. Generally, the unit dose to be administered has a radioactivity of about 0.01 mCi to about 100 mCi, preferably 1 mCi to 20 mCi. The composition to be injected at unit dosage is from about 0.01 ml to about 10 ml. After intravenous administration of Tc-99m labeled compositions, imaging of the organ or pathogenicity *in vivo* can take place as a matter of a few minutes. However, imaging can take place, if desired, in hours or even longer, after injecting into patients. In most instances, a sufficient amount of the administered dose will accumulate in the area to be imaged within about 0.5 of an hour to permit the taking of scintigraphic images. Any conventional method of scintigraphic imaging for diagnostic purposes can be utilized in accordance with this invention.

In yet another aspect of the present invention, kits for preparing the scintigraphic imaging agents of the invention are also provided. The kits comprise a first sealed vial containing a predetermined quantity of an unlabeled reagent of the invention and a sufficient amount of reducing agent to label the reagent with the radionuclide, e.g. Tc-99m, and a second sealed vial containing a predetermined quantity of a polysulfated glycan of the invention. Radiolabeled compositions of the invention are then made by labeling the contents of the first vial with the radionuclide and then mixing the contents of the first vial with the contents of the second vial to provide the composition ready for administration. Alternatively, appropriate amounts of the unlabeled reagent and the polysulfated glycan can be contained in a single vial. In preferred embodiments, the polysulfated glycan is dermatan sulfate or dermatan disulfate, or derivatives or mixtures thereof, although other polysulfated glycans may also be used.

In yet another aspect, the invention provides methods for using the radiolabeled, preferably Tc-99m labeled, scintigraphic imaging agent of the present invention for imaging sites of pathology, such as inflammation and infection, within a mammalian body by obtaining *in vivo* gamma scintigraphic images. A preferred method comprises the steps of administering an effective diagnostic amount of a radiolabeled scintigraphic imaging agent or a pharmaceutical composition of the invention and detecting the gamma radiation emitted by the radiolabel localized at the site to be

detected within the mammalian body. All of the aforementioned reagents, compositions and scintigraphic imaging agents may be used in this method, including the preferred embodiments as described above.

An alternative method is also provided for specifically radiolabeling whole blood and using mixtures comprising radiolabeled whole blood to image sites of inflammation within a mammalian body. In this aspect of the invention, the method comprises the steps of mixing whole blood with an amount, preferably from about 1 microgram to 100 milligrams, of a polysulfated glycan to form a mixture. A radiolabeled whole blood mixture is then formed by adding an amount, from about 1 microgram to 100 milligrams, of a radiolabeled, preferably Tc-99m labeled, composition that is a reagent comprising a polybasic moiety covalently linked to a radiolabel binding moiety, to the first whole blood mixture. Alternatively, the polysulfated glycan may first be added to the radiolabeled reagent of the invention and the resulting composition be added to form the radiolabeled whole blood mixture. This radiolabeled whole blood mixture is then administered to an animal such as a human being having or suspected of having a site of inflammation or infection *in vivo,* and the radioactive signal detected to localize the site of inflammation or infection as described herein. The requirement for antigenically-compatible, preferably but not necessarily autologous, heparinized whole blood to be used in this embodiment of the invention will be understood by those skilled in the art. The method provides an advantage over methods for labeling leukocytes known in the prior art, since the instant method eliminates the need for isolation of leukocytes from whole blood and attendant extensive *ex corpora* manipulation of whole blood.

The methods for making, labeling and using these compositions are more fully illustrated in the following Examples. Specific preferred embodiments of the present invention will become evident from the following more detailed description of the Examples and from the claims. The Examples are shown by way of illustration and not by way of limitation and the entire disclosure of all applications, patents, and publications, cited above and below is hereby incorporated by reference.

**Example 1: Preparation of Tc-99m P483H**

Typically, a vial from the kit for the preparation of Tc-99m P483 (143 μg of P483 peptide) was reconstituted with approximately 20 mCi of sodium pertechnetate Tc-99m Injection Solution in 1 ml total volume. The vial was incubated in a boiling water bath for 10 minutes after which time it was allowed to cool to room temperature. Three USP units of heparin sodium from a Lock-flush syringe were added to Tc-99m P483 and the mixture swirled gently. The vial was stored at room temperature for 10 minutes before quality control analysis was performed by ITLC.

Example 2: Preparation of Tc-99m P1827H, Tc-99m P1828H, Tc-99m 1829H, Tc-99m P2007H, and Tc-99m P2017H

A 150 μg portion of each peptide (as an anhydrous, counter-ion free peptide) in 0.9% saline (150 μL volume) was added to the Placebo Kit for the Preparation of Tc-99m P483. Approximately 20 mCi of Sodium Pertechnetate Tc-99m Injection Solution in 0.85 mL was added to the vial to obtain a 1 mL total volume. Each vial was incubated in a boiling water bath for 10 minutes after which time it was allowed to cool to room temperature. Three USP units of heparin sodium from a Lock-Flush® syringe were added to the respective Tc-99m peptide and the mixtures swirled gently. The vial was stored at room temperature for 10 minutes before quality control analysis was performed by ITLC.

Example 3: Preparation of Tc-99m Peptide DS or DDS

The peptides were radiolabeled as described above except that DS or DDS were added to the vial in place of heparin as indicated in Table 2. Briefly, a 1 mg/mL solution of either DS or DDS was prepared by dissolving solid DS or DDS in 0.9% saline. After addition of an appropriate amount of DS or DDS, the resulting solution of Tc-99m Peptide-DS or -DDS was swirled and stored at room temperature for 10 minutes prior to quality control analysis by ITLC.

**Example 4: Quality Control Analysis**

The analysis for determining the radiochemical purity of Tc-99m Peptide-H, -DS or -DDS (Tc-99m PGC's)) was performed by radiometric ITLC. Three saturator pad strips (2 cm x 11 cm) were spotted with approximately 10 μL of sample and one strip each developed in IPW (isopropanol:water, 1:1, v/v), 5% SDS solution, and AAA (3:3:1 Acetonitrile:glacial acetc acid:(1M ammonium acetate : methanol 1:1)). After the solvent had eluted to the top, the strips were cut at $R_f$= 0.75 (IPW), $R_f$= 0.20 (SDS) and $R_f$ = 0.50 (AAA). The radiochemical purity (RCP) was calculated from IPW and SDS strips, whereas the AAA strips yielded the distribution ratio (DR). The fraction containing free Tc-99m pertechnetate, Tc-99m edetate and Tc-99m glucoheptonate is located in the top 2 cm of the "IPW" strip, whereas the fraction containing Tc-99m non-peptide products (i.e. "non-mobile" impurities) that do not move with the solvent in the ITLC, is located in the bottom 3cm of the "S" strip.

$$\frac{\text{activity in the top 2 cm section (IPW)}}{\text{total activity in both parts of the "I" strip (IPW)}} \times 100\% = A$$

$$\frac{\text{activity in the bottom 3 cm section (SDS)}}{\text{total activity in both parts of the "S" strip (SDS);}} \times 100\% = B$$

% Labeling Efficiency (RCP) = 100% - % Total Sample Impurities (A+B);

$$\frac{\text{activity in the bottom section (AAA)}}{\text{activity in the top section (AAA)}} = \text{Distribution ratio}$$

**Example 5: The *E. coli* Infection Method**

*E. coli* organisms are serially passaged on sheep blood agar plates during 24 hour cycles to assure the organisms are in a growth-phase. Normal adult (2-2.5 kg) New Zealand White (NZW) rabbits are administered approximately $10^8$ freshly cultured *E. coli* organisms in 1 mL of normal saline (1.35 OD @ 600 nm) into the left calf. The inoculation of bacteria is done 18-24 hours prior to the injection of Tc-99m Peptide-H, -DS, or -DDS. Rabbits exhibit elevated body temperature and reactive hind leg retraction after 18-24 hours as hallmark of active infection.

**Example 6: Injection of Tc-99m PGC's**

A 500 µL portion of Tc-99m PGC's (Tc-99m Peptide-H, -DS, or -DDS) is diluted in 4.5 mL of 0.9% saline. For each animal, a 1 mCi dose is withdrawn in a weighed disposable syringe and the entire amount of Tc-99m PGC's is injected intravenously into the ear. The rabbits are left undisturbed until imaging time.

**Example 7: Imaging Protocols**

Four hours after injection of Tc-99m PGC, the animals received a lethal intravenous injection of barbiturate (Euthasol®. Each animal is placed on the face of the gamma camera (inverted camera) for anterior views. A low energy/high efficiency collimator is preferred. The Tc-99m window is opened to 20%. The image collection protocol includes either a count maximum of 500 kilocounts or a time maximum of 300 seconds, whichever occurs first.

A terminal blood sample is collected by cardiac puncture. Tissues are harvested for determination of percent injected dose (%ID) and percent injected dose per gram (%ID/g). Tissues include terminal blood, thyroid, palate, salivary glands, lungs, liver, kidneys, spleen, gastrointestinal tract, bladder and expelled urine (pads), infected muscle (n = 6 x 1 g samples) and contralateral (control) muscle (n = 2 x 1 g samples).

**Example 8: Biodistribution**

Biodistribution (see Table 3) is presented as %ID and %ID/g as well as by the image contrast ratios $I_{max}$:B (infected muscle versus terminal blood) and $I_{max}$:C (infected muscle versus control muscle).

**Example 9: Effect of Lysine Residues**

The binding of PF4 derivative peptides to polysulfated glycans is purported to be due to ionic interactions between lysines and sulfate groups. To increase the binding of heparin to P483, the lysine residues were substituted with arginine residues which are known to bind to polysulfated glycans with higher affinity. One measurement value for Tc-99m PGC formation is the distribution ratio (DR, see Quality Control Example); a higher DR indicates enhanced binding of the polysulfated glycan to the peptide. As shown in Table 2, the DR for Tc-99m P483H was 0.68. When arginine residues were substituted. for the lysine residues, the DR was substantially higher (DR = 0.86 for Tc-99m P1827H) suggesting stronger PGC complex formation for Tc-99m P1827H than for Tc-99m P483H. The higher binding affinity of polysulfated glycans to Tc-99m P1827 also translated into a more favorable biodistribution (Table 3). Even though infection uptake was comparable for Tc-99m P1827H and Tc-99m P483H, the image contrast values $I_{max}$:C and $I_{max}$:B were significantly better for Tc-99m P1827H ($I_{max}$:C = 44 and $I_{max}$:B = 4.3) than for Tc-99m P483H ($I_{max}$:C = 18 and $I_{max}$:B = 2.4). There was no significant improvement over Tc-99m P1827H either when only lysine residues in the N-terminus pentalysine sequence were replaced with arginine (P1828) or when only lysines in the PF4 region were replaced by arginines (P1829). Thus, substitution of Lys residues with Arg residues resulted in higher image contrast values, lower palate and thyroid accumulation, and higher lung to liver ratio ( see also Figure 1).

**Table 1. Amino Acid Sequence of Peptides Tested**

| Code | Amino Acid Sequence* |
|---|---|
| P483 | Ac-KKKKKCGCGGPLYKKIIKKLLES (SEQ ID No. 1) |
| P1827 | Ac-RRRRRCGCGGPLYRRIIRRLLES (SEQ ID No. 3) |
| P1828 | Ac-RRRRRCGCGGPLYKKIIKKLLES (SEQ ID No. 4) |
| P1829 | Ac-KKKKKCGCGGPLYRRIIRRLLES (SEQ ID No. 5) |
| P2007 | Ac-KKKKKPenGCGGPLYKKIIKKLLES (SEQ ID No. 6) |
| P2017 | Ac-KKKKKC$_{iso}$GC$_{iso}$GGPLYKKIIKKLLES (SEQ ID No. 7) |

*The amino acids are represented by their single letter codes. Ac = Acetyl; Pen = Penicillamine; C$_{iso}$ = Isocysteine

Table 2.

| Preparation and Radiochemical Purity Data | | | | |
|---|---|---|---|---|
| Peptide (150µg) | Polysulfated Glycan (aAmount) | Imaging Agent Code (Tc-99m PGC) | %RCP | DR |
| P483 | Heparin Sodium (3U) | Tc-99m P483H | 95% | 0.68 |
| P1827 | Heparin Sodium (3U) | Tc-99m P1827H | 95% | 0.86 |
| P2007 | Heparin Sodium (3U) | Tc-99m P2007H | 95% | 0.20 |
| P2017 | Heparin Sodium (3U) | Tc-99m P2017H | 95% | 0.25 |
| P483 | Dermatan Sulfate (60 µg) | Tc-99m P483DS | 94% | 0.51 |
| P483 | Dermatan Disulfate (75 µg) | Tc-99m P483DDS | 93% | 0.54 |
| P1827 | Dermatan Sulfate (60 µg) | Tc-99m P1827DS | 94% | 12.9 |
| P1827 | Dermatan Disulfate (50 µg) | Tc-99m P1827DDS | 93% | 1.60 |

Table 3.

| Summary of Infection Uptake and Biodistribution (%ID/g and Ratios) | | | | | | | |
|---|---|---|---|---|---|---|---|
| Code (Tc-99m PGC) | $I_{max}$ (%ID/g) | $I_{avg}$ (%ID/g) | Palate (%ID/g) | Thyroid (%ID/g) | Lung:Liver (%ID/g) | $I_{max}$:C | $I_{max}$:B |
| Tc-99m P483H | 0.05 | 0.04 | 0.36 | 0.45 | 0.90 | 18 | 2.4 |
| Tc-99m P1827H | 0.06 | 0.04 | 0.06 | 0.02 | 2.10 | 44 | 4.3 |
| Tc-99m P2007H | 0.02 | 0.02 | 0.05 | 0.05 | 2.17 | 12 | 1.9 |

Table 3. (continued)

| Summary of Infection Uptake and Biodistribution (%ID/g and Ratios) | | | | | | | |
|---|---|---|---|---|---|---|---|
| Code (Tc-99m PGC) | $I_{max}$ (%ID/g) | $I_{avg}$ (%ID/g) | Palate (%ID/g) | Thyroid (%ID/g) | Lung:Liver (%ID/g) | $I_{max}$:C | $I_{max}$:B |
| Tc-99m P2017H | 0.03 | 0.02 | 0.04 | 0.07 | 1.25 | 29 | 2.9 |
| Tc-99m P483DS | 0.09 | 0.06 | 0.31 | 0.48 | 5.68 | 40 | 3.7 |
| Tc-99m P1827DS (100µg DS) | 0.331 | 0.25 | 0.036 | 0.031 | 5.9 | 165 | 27.6 |
| Tc-99m P483DDS | 0.11 | 0.08 | 0.15 | 0.13 | 2.86 | 56 | 4.9 |
| Tc-99m P1827DDS | 0.11 | 0.07 | 0.10 | 0.20 | 2.60 | 101 | 9.4 |

**Example 10: Effect of Chelator**

P2007 and P2017 are two P483 analogs in which the Cys-Gly-Cys chelator has been replaced with *iso*Cys-Gly-*iso*Cys and Pen-Gly-Cys, respectively. These peptides were radiolabeled with Tc-99m to > 95% RCP and, with heparin, formed PHC's. Tc-99m P2007H and Tc-99m P2017H showed lower uptake in thyroid when compared to Tc-99m P483H (Table 3, Figure 2) in the rabbit infection model (an advantage), but infection imaging parameters were unaffected by the change in Tc-99m chelator (Figure 2) from a Cys-Gly-Cys to Pen-Gly-Cys or *iso*Cys-Gly-*iso*Cys ligand.

**Example 11: Effect of Polysulfated Glycan**

Heparin is a polydisperse mixture of variably sulfated glycans. In contrast, dermatan sulfate is a homogeneous linear chain polysulfated glycan with a single repeating disaccharide unit. (The level of sulfation of dermatan sulfate is comparable with heparin. By hypersulfating DS at the 6-position, dermatan disulfate is formed. Dermatan disulfate has all the structural/physiochemical properties of DS but it contains two sulfate groups per disaccharide unit rather than one as in DS. Peptides containing lysine (P483, P2007, P2017) or arginine (P1827, P1828, P1829) residues are proposed to bind to polysulfated glycans via amine or guanidine-to-sulfate ionic interactions (Hilemann, R.E. et al. (1998) *Bioessays,* 20:156-167). Similar binding interaction is envisioned for these peptides with DS and DDS. Tc-99m P483 and Tc-99m P1827 form complexes with DS and DDS (Table 2). P1827 would be expected to bind to DDS and DS with higher affinity than P483 analogous to their heparin binding characteristics. Tc-99m P1827DDS (DR = 1.6) and P1827DS (DR = 10.5) show a higher DR than P1827H (DR = 0.86) indicating a higher degree of formation of PGC's.

The change of the polysulfated glycan from heparin to DS to DDS resulted in clear differences in the values of infection uptake and biodistribution. Tc-99m P483DS exhibited similar %ID/g in palate and thyroid as Tc-99m P483H in a rabbit infection model (Table 3). Thyroid uptake is diminished upon using DDS with Tc-99m P483.

The image-contrast ratios of $I_{max}$:C and $I_{max}$:B show a dramatic increase when the polysulfated glycan is well-defined, homogenous and sulfate-rich entity like DS or DDS (Table 3) compared to heparin. The contrast value $I_{max}$:C gradually increases from H ($I_{max}$:C =18) to DS ($I_{max}$:C = 40) to DDS ($I_{max}$:C =56) for Tc-99m P483 PGC's (Figure 3). In the case of Tc-99m P1827, the contrast value $I_{max}$:C also increased from H ($I_{max}$:C = 4.3) to DS ($I_{max}$:C = 165) to DDS ($I_{max}$:C = 101).

The infection uptake and contrast value is further dependent on the peptide-to-polysulfated glycan ratio. For the peptide P1827 and the polysulfated glycan dermatan sulfate, the optimal weight ratio between the two components was determined. A P1827/DS ratio of 1.5:1 (w/w) results in the highest uptake at the infection site and the highest contrast values for Tc-99m P1827DS (see Table 4).

| Table 4: Dependence of Infection Uptake (%ID/g and Image Contrast Ratios) of Tc-99m P1827DS on the Peptide / Polysulfated Glycan weight ratio | | | | |
|---|---|---|---|---|
| **Amount of DS added to 150 µg P1827** | **$I_{max}$ (%ID/g)** | **$I_{avg}$ (%ID/g)** | **$I_{max}$:C** | **$I_{max}$:B** |
| 10 µg | 0.036 | 0.026 | 26.5 | 0.7 |
| 30 µg | 0.083 | 0.062 | 62.5 | 5.2 |
| 60 µg | 0.088 | 0.058 | 66.5 | 2.0 |
| 100 µg | 0.331 | 0.25 | 165 | 27.6 |
| 200 µg | 0.184 | 0.128 | 184 | 11.5 |
| 1000 µg | 0.029 | 0.021 | 19.2 | 2.4 |

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications to adapt it to various usages and conditions.

## Description of the Drawings

FIG. 1 illustrates a comparison of the infection uptake and biodistribution of Tc-99m P483H to Tc-99m P1827H in the *E. coli* rabbit infection model.

FIG. 2 illustrates a comparison of the infection uptake and biodistribution of Tc-99m 2017H toTc-99m P483H in the *E. coli* rabbit infection model.

FIG. 3 illustrates a comparison of the infection uptake and biodistribution of Tc-99m P483H to Tc-99m P483DS and Tc-99m 483DDS in the *E. coli* rabbit infection model.

FIG. 4 illustrates a comparison of the infection uptake and biodistribution of Tc-99m P1827H to Tc-99m P1827DDS in the *E. coli* rabbit infection model.

## Claims

**1.** A reagent comprising:

i) a polybasic compound comprising a peptide, wherein the peptide comprises at least four arginine residues; and

ii) a radiolabel-binding moiety covalently linked to the polybasic compound;

wherein the reagent is capable of accumulating at sites of pathology in the body.

**2.** The reagent of claim 1, wherein the peptide has from about 5 to about 100 amino acids.

**3.** The reagent of claim 1 or claim 2, wherein the reagent is capable of accumulating at sites of inflammation or infection *in vivo.*

**4.** The reagent of any of claims 1 to 3, wherein the peptide comprises an amino acid sequence corresponding to a sequence of about 5 to 70, preferably about 50, 40, 30, 20, 15, 14, 13, 12, 11, 10, or 9 contiguous amino acids of human Platelet Factor 4, or having at least 40, 50, 60, 70, 80, 90, 95, 96, 97, 98, or 99% sequence identity to said

sequence.

**5.** The reagent of claim 4, wherein said sequence of contiguous amino acids is from the C-terminus of human Platelet Factor 4 (PF4).

**6.** The reagent of claim 4 or claim 5, wherein the at least four arginine residues of the polybasic compound represent a substitution of corresponding lysine residues in the amino acid sequence of human Platelet Factor 4, or represent an addition to the amino acid sequence corresponding to said sequence of human Platelet Factor 4.

**7.** The reagent of any of claims 1 to 6, wherein the polybasic compound comprises from 4 to 9, preferably five, six, seven, eight and most preferably nine arginine residues.

**8.** The reagent of any of the preceding claims, wherein the radiolabel-binding moiety is selected from the group consisting of:

I.

Cp(aa)Cp

wherein Cp is a cysteine having a protected or unprotected thiol group and (aa) is an amino acid; or

II.

a radiolabel-binding moiety comprising a single thiol moiety, wherein the single thiol moiety has a formula:

$$A\text{-}CZ(B)\text{-}[C(R^1R^2)_n\text{-}X$$

wherein A is H, HOOC, $H_2NOC$, (peptide)-NHOC, (peptide)-OOC or $R^4$;
B is H, SH, $-NHR^3$, $-N(R^3)$-(peptide), or $R^4$;
X is H, SH, $-NHR^3$, $-N(R^3)$-(peptide) or $R^4$;
Z is H or $R^4$;
$R^1$, $R^2$, $R^3$ and $R^4$ are independently H or lower straight or branched chain or cyclic alkyl;
n is 0, 1 or 2;
and where B is $-NHR^3$ or $-N(R^3)$-(peptide), X is SH, and n is 1 or 2;
where X is $NHR^3$ or $-N(R^3)$-(peptide), B is SH, and n is 1 or 2;
where B is H or $R^4$, A is HOOC, $H_2NOC$, (peptide)-NHOC, or (peptide)-OOC, X is SH, and n is 0 or 1;
where A is H or $R^4$, then where B is SH, X is $-NHR^3$ or $-N(R^3)$-(peptide) and where X is SH, B is $NHR^3$ or $-N(R^3)$-(peptide);
where X is H or $R^4$, A is HOOC, $H_2NOC$, (peptide)-NHOC, or (peptide)-OOC and B is SH;
where Z is methyl, X is methyl, A is HOOC, $H_2NOC$, (peptide)-NHOC, or (peptide)-OOC, B is SH and n is 0;
and wherein the thiol moiety is in the reduced form;

III.

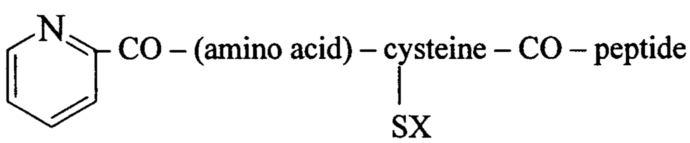

wherein X = H or a protecting group;
(amino acid) = any amino acid;
or

IV.

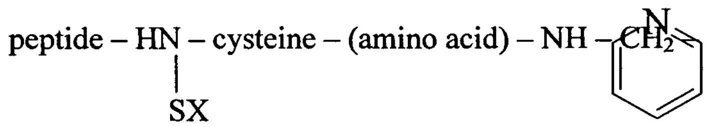

wherein X = H or a protecting group;
(amino acid) = any amino acid;
or

V.

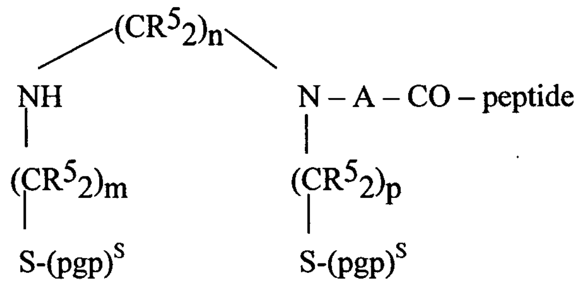

wherein each $R^5$ is independently H, lower alkyl, phenyl, or phenyl substituted with lower alkyl or lower alkoxy;
each $(pgp)^s$ is independently a thiol protecting group or H;
m, n and p are independently 2 or 3;
A = linear or cyclic lower alkyl, aryl, heterocyclyl, combinations or substituted derivatives thereof;
or

VI.

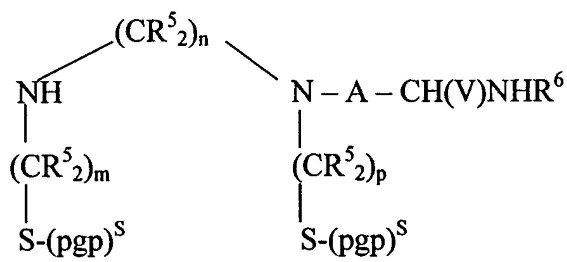

wherein each $R^5$ is independently H, lower alkyl, phenyl, or phenyl substituted with lower alkyl or lower alkoxy;
each $(pgp)^s$ is independently a thiol protecting group or H;
m, n and p are independently 1, 2 or 3;
A = linear or cyclic lower alkyl, aryl, heterocyclyl, or combinations or substituted derivatives thereof;
V = H or -CO-peptide;
$R^6$ = H or peptide;
and wherein when V = H, $R^6$ = peptide and when $R^6$ = H, V = -CO-peptide.

**9.** The reagent of claim 8, wherein the radiolabel-binding moiety is Cp(aa)Cp and Cp is a protected cysteine having a protecting group of formula:

$$-CH_2\text{-NH-CO-R}$$

wherein R is a lower alkyl, 2-pyridyl, 3-pyridyl, 4-pyridyl, phenyl, or phenyl substituted with lower alkyl, hydroxy, lower alkoxy, carboxy, or lower alkoxycarbonyl.

**10.** The reagent of claim 9, wherein the radiolabel-binding moiety has the formula:

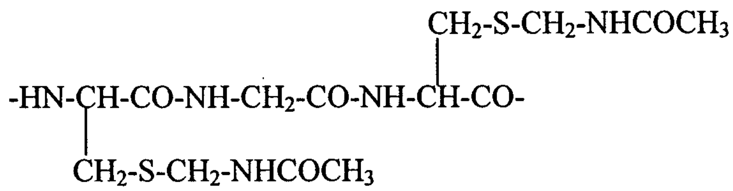

**11.** The reagent of any of the preceding claims, wherein the polybasic compound and the radiolabel-binding moiety are covalently linked through from about one to about twenty amino acids.

**12.** The reagent of claim 11, wherein the amino acid covalently linking the polybasic compound and the radiolabel-binding moiety is one or more glycines.

**13.** The reagent of any of the preceding claims, wherein the reagent comprises the amino acid sequence

KKKKKCGCGGPLYKKIIKKLLES (SEQ ID No. 2),

except that at least four, preferably five, six, seven, eight and most preferably nine of the lysine residues of said peptide are substituted by arginine residues.

**14.** The reagent of any of the preceding claims, wherein the polybasic compound and the radiolabel-binding moiety covalently linked thereto together form a peptide having an amino acid sequence selected from the group consisting of:

Acetyl-RRRRRCGCGGPLYRRIIRRLLES (SEQ ID No. 3);

Acetyl-RRRRRCGCGGPLYKKIIKKLLES (SEQ ID No. 4);

and

Acetyl-KKKKKCGCGGPLYRRIIRRLLES(SEQ ID No. 5).

**15.** The reagent of any of the preceding claims, wherein the polybasic compound and the radiolabel-binding moiety covalently linked thereto together form a peptide having the amino acid sequence:

Acetyl-RRRRRCGCGGPLYRRIIRRLLES (SEQ ID No. 3).

**16.** A multimeric reagent comprising

i) at least two polybasic compounds as defined in any of the preceding claims which may be the same or different;

ii) at least one radiolabel-binding moiety as defined in any of the preceding claims covalently linked to at least one of the polybasic compounds; and

iii) a polyvalent linker moiety covalently linked to the polybasic compounds, the radiolabel-binding moieties or both;

wherein the molecular weight of the multimeric polyvalent reagent is less than about 20,000 Da.

**17.** The multimeric reagent of claim 16, wherein the polyvalent linking moiety is comprised of at least 2 linker functional groups capable of covalently bonding to the polybasic compounds or the radiolabel-binding moieties, preferably wherein at least 2 of the linker functional groups are identical; optionally wherein the linker functional groups are primary or secondary amines, hydroxyl groups, carboxylic acid groups or thiol-reactive groups, the thiol-reactive groups being selected from maleimido groups and chloroacetyl, bromoacetyl and iodoacetyl groups.

**18.** The multimeric reagent of claim 16 or claim 17, wherein the polyvalent linker is selected from the group consisting of:

*bis*-succinimidylmethylether;

4-(2,2-dimethylacetyl)benzoic acid;

*tris*(succinimidylethyl)amine;

*bis*-succinimidohexane;

4-(O-$H_2$CO-Gly-Gly-Cys.amide)acetophenone;

*tris*(acetamidoethyl)amine;

*bis*(acetamidomethyl)amine;

*bis*(acetamidoethyl)amine;

$\alpha,\varepsilon$-bis(acetyl)lysine;

lysine; and

1,8-*bis*-acetamido-3,6-dioxa-octane;

or a derivative of any of the above-listed polyvalent linkers.

**19.** A complex formed by either, (a) reacting a reagent as defmed in any of claims 1 to 18 with technetium-99m in the presence of a reducing agent, preferably a reducing agent selected from the group consisting of a dithionite ion, a stannous ion, and a ferrous ion, or (b) labeling the reagent as defined in any of claims 1 to 18 with technetium-99m by ligand exchange of a prereduced technetium-99m complex.

**20.** A composition comprising

(a) the reagent as defined in any of claims 1 to 18

(b) a polysulfated glycan having a molecular weight of at least about 1000 Da;

wherein the composition is capable of accumulating at sites of pathology in the mammalian body.

**21.** The composition of claim 20, wherein the polysulfated glycan is dextran sulfate, chondroitin sulfate, dermatan sulfate or dermatan disulfate, or any derivative or mixture thereof, preferably wherein the polysulfated glycan is dermatan sulfate or dermatan disulfate.

**22.** The composition of claim 20 or claim 21, wherein the (w/w) ratio of the polybasic compound to the polysulfated glycan is from 0.1:1 to 20:1, preferably from 0.2:1 to 10:1, more preferably from 0.5:1 to 5:1 or 1:1 to 2:1, and is most preferably about 1.45:1 or 1.5:1.

**23.** The composition of any of claims 20 to 22, wherein the composition is capable of accumulating at sites of inflammation or infection *in vivo.*

**24.** The composition of any of claims 20 to 23, wherein the composition is capable of achieving an image contrast ratio $I_{max}$:C between muscle tissue infected by *E. coli* and uninfected muscle tissue in the rabbit injection model of more than 25, preferably more than 40, and most preferably more than 60, and / or wherein the composition is capable of achieving an image contrast ratio $I_{max}$:B between muscle tissue infected by *E. coli* and terminal blood in the rabbit injection model of more than 3, preferably more than 4, 5, 6, 7, or 8 and most preferably more than 9;
when the reagent of the composition is labeled with Tc-99m and administered together with the polysulfated glycan.

**25.** The composition of any of claims 20 to 24, wherein the reagent is a peptide having the sequence

Acetyl-RRRRRCGCGGPLYRRIIRRLLES (SEQ ID No. 3),

and wherein the polysulfated glycan is dermatan sulfate.

**26.** A scintigraphic imaging agent comprising

(a) the composition of any of claims 20 to 25; and

(b) a radioisotope,

wherein the radioisotope is complexed to the reagent within the composition via its radiolabel-binding moiety.

**27.** The scintigraphic imaging agent of claim 26, wherein the radioisotope is selected from the group consisting of technetium-99m, fluor-18, gallium-67, gallium-68, indium-111, iodine-123, iodine-125, ytterbium-169, or rhenium-186.

**28.** The scintigraphic imaging agent of claim 26 or claim 27, wherein the radioisotope is technetium-99m.

**29.** The scintigraphic imaging agent of any of claims 26 to 28, wherein the imaging agent achieves an image contrast ratio $I_{max}$:C between muscle tissue infected by Escherichia coli and uninfected muscle tissue in the rabbit injection model of more than 25, preferably more than 40, and most preferably more than 60, and / or wherein the imaging agent achieves an image contrast ratio $I_{max}$:13 between muscle tissue infected by *E. coli* and terminal blood in the rabbit injection model of more than 3, preferably more than 4, 5, 6, 7, or 8 and most preferably more than 9.

**30.** A pharmaceutical composition comprising the reagent as defined in any of claims 1 to 18, the complex as defined in claim 19, the composition as defined in any of claims 20 to 25, or the scintigraphic imaging agent as defined in any of claims 26 to 29, further comprising a pharmaceutically acceptable carrier.

**31.** The reagent of any of claims 1 to 18, the complex of claim 19, the composition of any of claims 20 to 25, the scintigraphic imaging agent of any of claims 26 to 29, or the pharmaceutical composition of claim 30 for use for imaging a site of pathology within a mammalian body.

**32.** The reagent, the complex, the composition, the scintigraphic imaging agent, or the pharmaceutical composition of claim 31, wherein the site to be imaged is a site of inflammation or infection.

**33.** Use of the reagent of any of claims 1 to 18, the complex of claim 19, the composition of any of claims 20 to 25,

the scintigraphic imaging agent of any of claims 26 to 29, or the pharmaceutical composition of claim 30 in the manufacture of a diagnostic pharmaceutical for imaging a site of pathology within a mammalian body.

**34.** The use according to claim 33, wherein the site to be imaged is a site of inflammation or infection.

**35.** A kit for preparing a radiopharmaceutical preparation, said kit comprising

(a) a first sealed vial containing

(i) a predetermined quantity of a reagent as defined in any of claims 1 to 18; and

(ii) a sufficient amount of a reducing agent to label the reagent with a radioisotope; and

(b) a second sealed vial containing a predetermined quantity of a polysulfated glycan as defined in any of claims 20 to 23.

**36.** The kit of claim 35, wherein the reducing agent is selected from the group consisting of a dithionite ion, a stannous ion, a ferrous ion.

**37.** The kit of claim 35 or claim 36, wherein the reagent has the formula:

Acetyl-RRRRRCGCGGPLYRRIIRRLLES (SEQ ID No. 3);

and
wherein the polysulfated glycan is dermatan sulfate.

**38.** The kit of any of claims 35 to 37, wherein the radioisotope is technetium-99m.

**39.** A process for preparing a reagent as defined in any of claims 1 to 18 by *in vitro* chemical synthesis.

**40.** The process of claim 39, wherein the reagent is prepared by solid phase peptide synthesis.

**41.** The process of claim 39 or claim 40, wherein the radiolabel-binding moiety is covalently linked to the peptide during solid phase peptide synthesis.

**42.** A method of imaging a site of pathology within a mammalian body comprising the steps of:

a) administering an effective diagnostic amount of a scintigraphic imaging agent as defined in any of the claims 26 to 29 or a pharmaceutical composition as defined in claim 30; and

b) detecting a radioactive signal from the radiolabel localized at said site.

**43.** The method according to claim 42, wherein the radiolabel is localized at a site of inflammation or infection.

**44.** The method according to claim 42 or claim 43, wherein the reagent is a peptide selected from the group consisting of:

Acetyl-RRRRRCGCGGPLYRRIIRRLLES (SEQ ID No. 3);

Acetyl-RRRRRCGCGGPLYKKIIKKLLES (SEQ ID No. 4);

and

Acetyl-KKKKKCGCGGPLYRRIIRRLLES (SEQ ID No. 5).

**45.** The method according to any of claims 42 to 44, wherein the polysulfated glycan is dermatan sulfate.

**46.** The method according to any of claims 42 to 45, wherein the radioisotope is technetium-99m.

**47.** A method of imaging a site of inflammation or infection within a mammalian body comprising the steps of:

(a) mixing whole blood and from about 1 microgram to 100 milligrams of the scintigraphic imaging agent of any of claims 26 to 29 or the pharmaceutical composition of claim 30;

(b) administering said mixture to a mammal; and

(c) detecting a radioactive signal from the radioisotope localized at said site.

**48.** The method of claim 47, wherein the radioisotope is technetium-99m.

**Figure 1: Infection Uptake and Biodistribution: Comparison of Tc-99m P483H to Tc-99m P1827H in the *E. coli* Rabbit Infection Model**

**A) Tc 99m P483H**

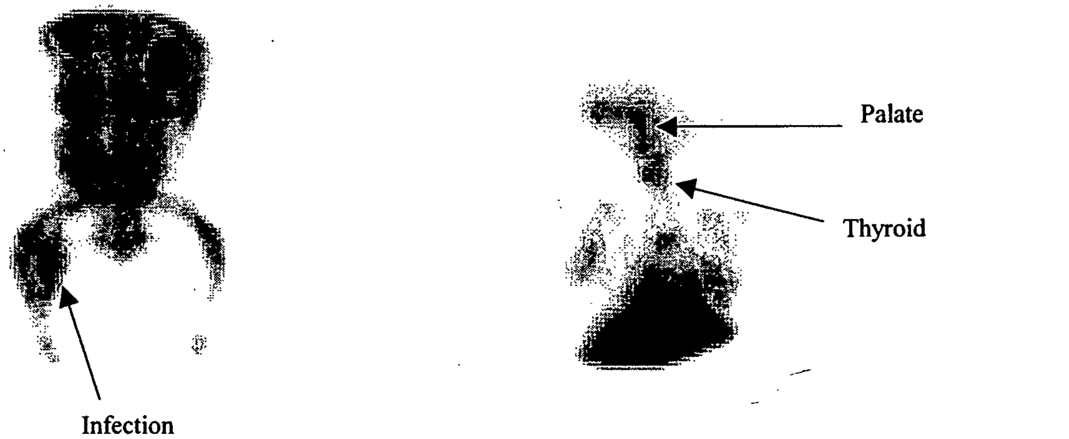


**B) Tc 99m P1827H**

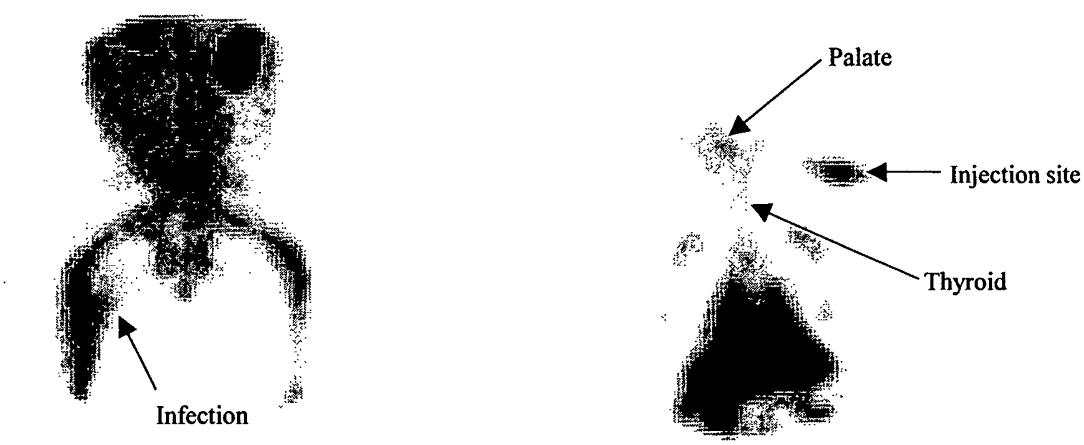

**Figure 2:** **Infection Uptake and Biodistribution: Comparison of Tc-99m P483H to Tc-99m P2017H in the *E. coli* Rabbit Infection Model**

**A) Tc 99m P483H**

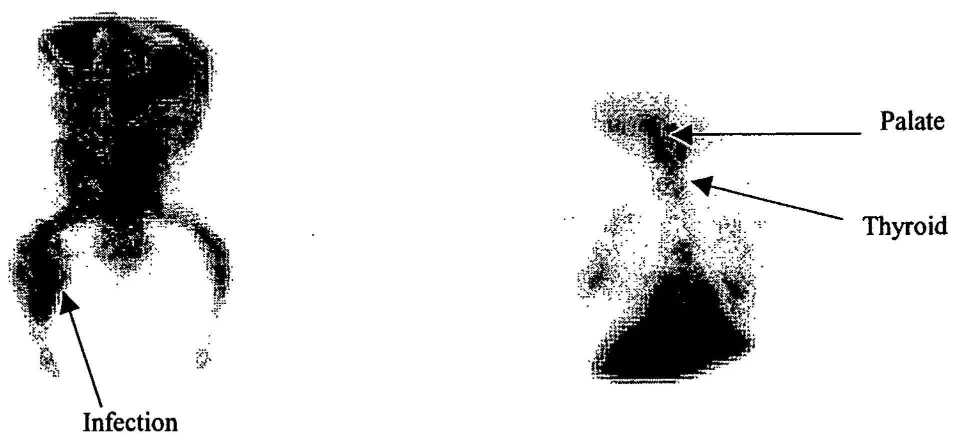


**B) Tc 99m 2017H**

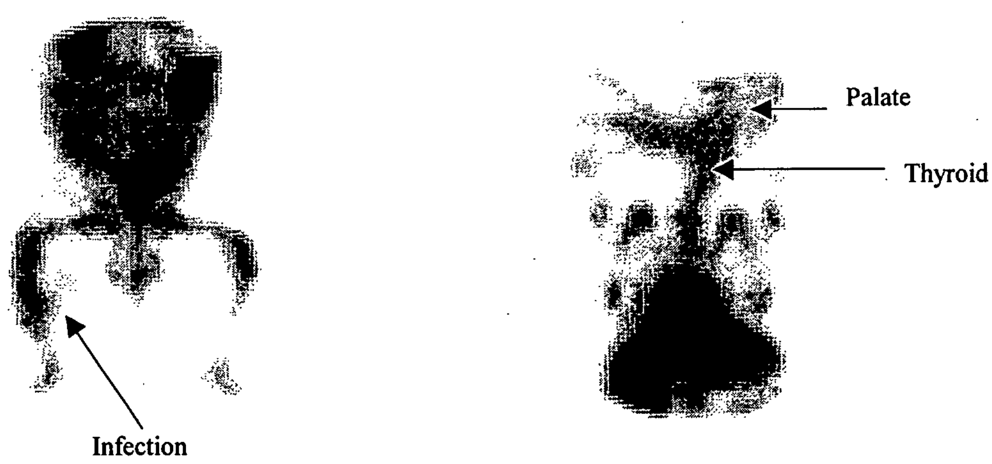

**Figure 3: Infection Uptake and Biodistribution: Comparison of Tc-99m P483H to Tc-99m P483DS / Tc-99m P483DDS in the *E. coli* Rabbit Infection Model**

**A) Tc 99m P483H**

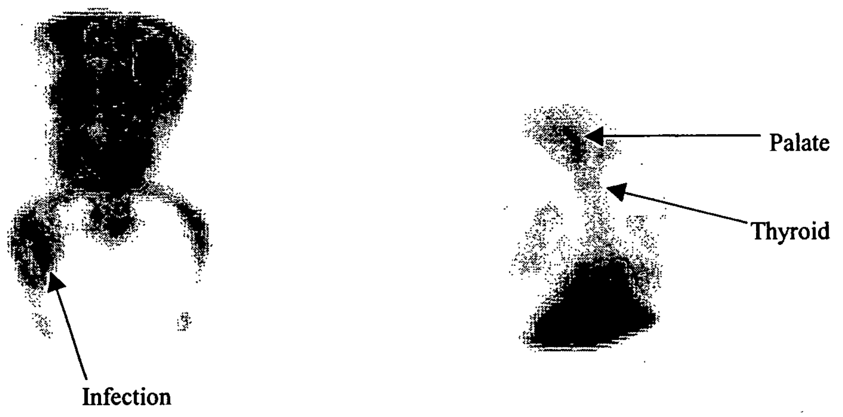


**B) Tc 99m P483DS**

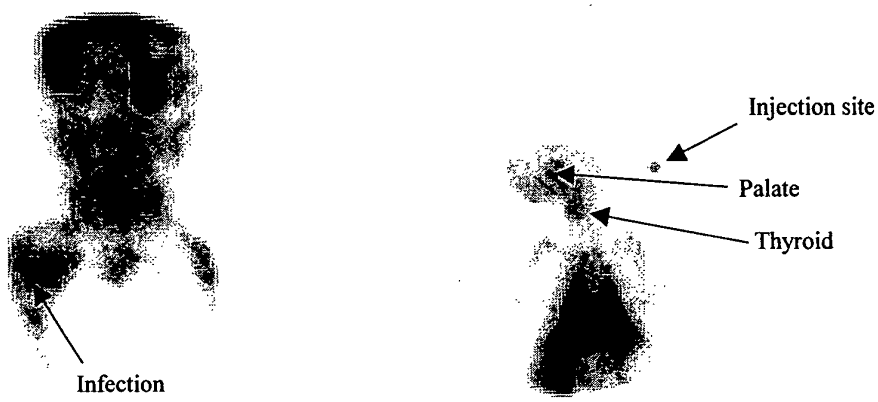


**C) Tc 99m P483DDS**

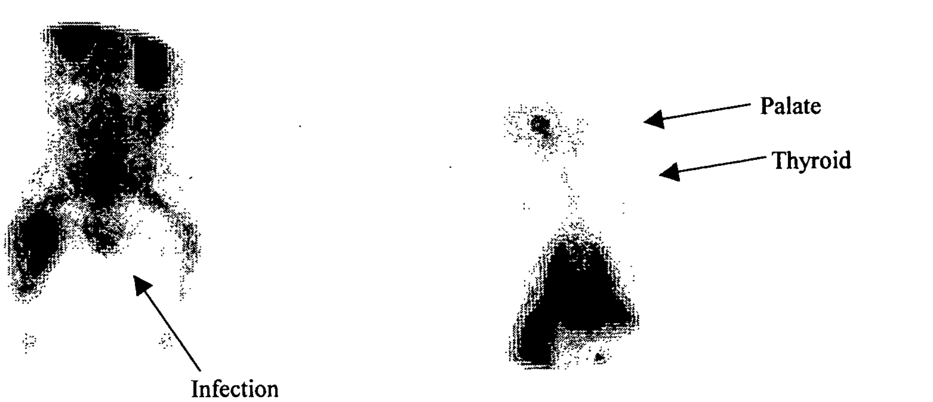

**Figure 4: Infection Uptake and Biodistribution: Comparison of Tc-99m P1827H to Tc-99m P1827DS / Tc-99m P1827DDS in the *E. coli* Rabbit Infection Model**

**A) Tc 99m P1827H**

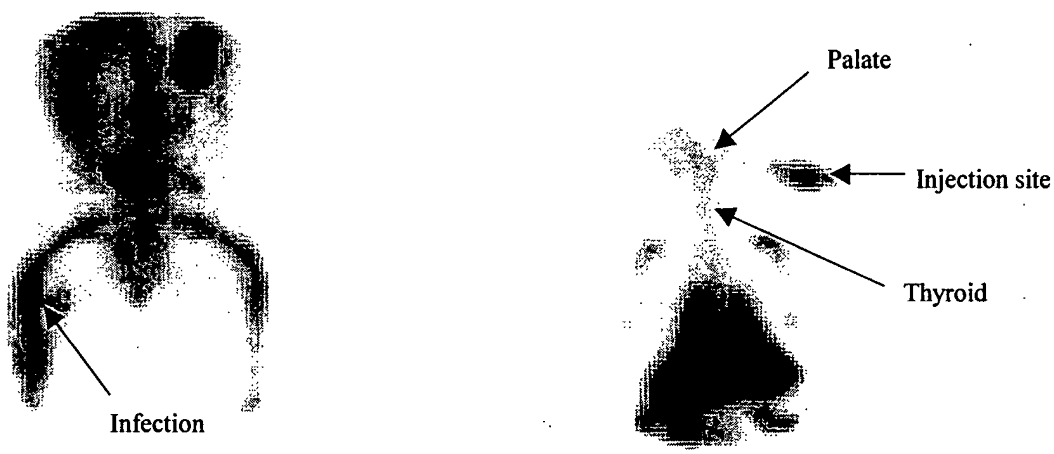


**B) Tc 99m 1827DS**

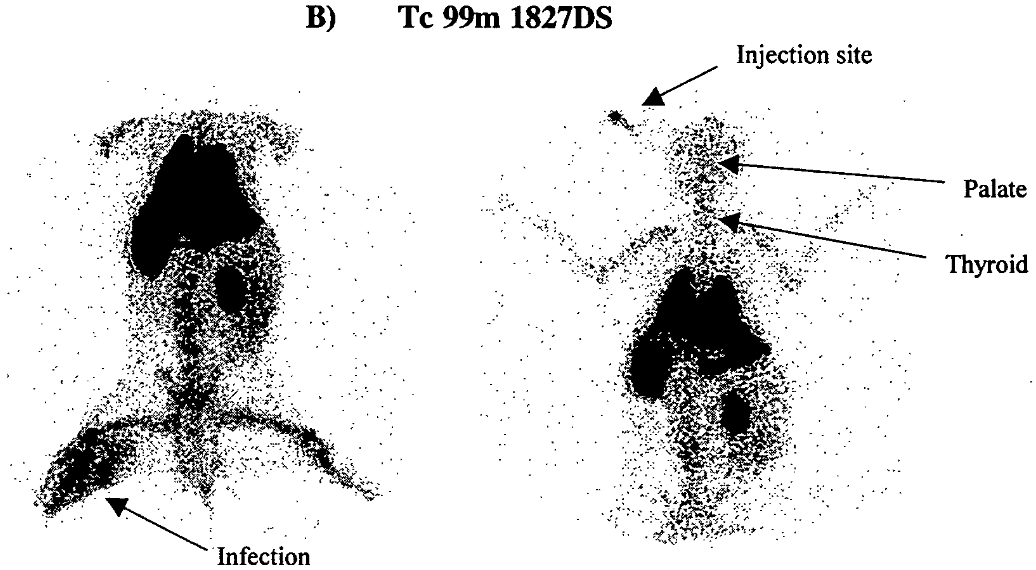

**C) Tc 99m P1827DDS**

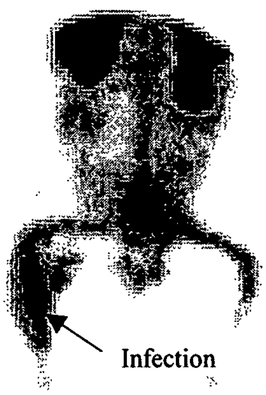


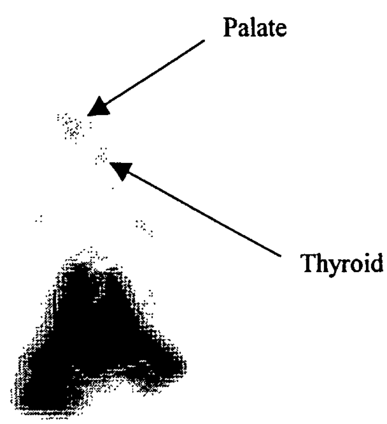

European Patent Office

# EUROPEAN SEARCH REPORT

Application Number
EP 03 00 0204

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 017 510 A (BUTTRAM SCOTT ET AL) 25 January 2000 (2000-01-25)<br>* the whole document *<br>--- | 1-4, 7-12,19, 26-34, 39-48 | A61K51/08 |
| X | US 5 605 671 A (STRIETER ROBERT M ET AL) 25 February 1997 (1997-02-25)<br>* the whole document *<br>--- | 1,2,4, 7-12, 16-19, 26-31, 33,39-48 | |
| X | US 5 346 686 A (LYLE LEON R ET AL) 13 September 1994 (1994-09-13)<br>* the whole document *<br>--- | 1-4, 7-12,19, 26-34, 39-48 | |
| X | WO 93 23085 A (LISTER JAMES JOHN ;DIATECH INC (US); DEAN RICHARD T (US)) 25 November 1993 (1993-11-25)<br>* the whole document *<br>--- | 1-4, 7-12,19, 26-34, 39-48 | TECHNICAL FIELDS SEARCHED (Int.Cl.7)<br>A61K |
| X | WO 00 20594 A (REIS LUIS F L ;PIRES EDUARDO G (BR); ABRANTES EDUARDO FERNANDES (B) 13 April 2000 (2000-04-13)<br>* sequences 2, 14, 16 *<br>* page 35 *<br>---<br>-/-- | 1-4, 7-12,19, 26-34, 39-48 | |

The present search report has been drawn up for all claims

| Place of search | Date of completion of the search | Examiner |
|---|---|---|
| MUNICH | 12 June 2003 | Bardili, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 00 0204

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | KNIGHT, L.C. ET AL.: "In vitro platelet binding compared with in vivo thrombus imaging using alpha(IIb)beta(3)-targeted radioligands" THROMBOSIS AND HAEMOSTASIS, vol. 80, no. 5, 1998, pages 845-851, XP009012157 * figures 1,3,4; table 1 * | 1,2,4, 30,31,33 | |
| A | MAYO, KEVIN H. ET AL: "Heparin binding to platelet factor-4. An NMR and site-directed mutagenesis study: arginine residues are crucial for binding" BIOCHEMICAL JOURNAL (1995), 312(2), 357-65 , 1995, XP009012158 | | |
| A,D | DEUEL, T.F. ET AL.: "Amino acid sequences of human platelet factor 4" PROC. NATL. ACAD. SCI., vol. 74, 1977, pages 2256-8, XP009012231 | | |

TECHNICAL FIELDS SEARCHED (Int.Cl.7)

The present search report has been drawn up for all claims

| Place of search | Date of completion of the search | Examiner |
|---|---|---|
| MUNICH | 12 June 2003 | Bardili, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 00 0204

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2003

| Patent document cited in search report | Publication date | Patent family member(s) | Publication date |
|---|---|---|---|
| US 6017510 A | 25-01-2000 | US 5711931 A | 27-01-1998 |
| | | US 5807538 A | 15-09-1998 |
| | | AT 237365 T | 15-05-2003 |
| | | AU 683015 B2 | 30-10-1997 |
| | | AU 4043893 A | 05-10-1993 |
| | | CA 2131816 A1 | 14-09-1993 |
| | | DE 69332879 D1 | 22-05-2003 |
| | | EP 0630265 A1 | 28-12-1994 |
| | | JP 7504902 T | 01-06-1995 |
| | | WO 9317719 A1 | 16-09-1993 |
| | | US 5989519 A | 23-11-1999 |
| | | US 5965107 A | 12-10-1999 |
| US 5605671 A | 25-02-1997 | US 5413778 A | 09-05-1995 |
| | | US 5346686 A | 13-09-1994 |
| | | AT 150977 T | 15-04-1997 |
| | | AU 674039 B2 | 05-12-1996 |
| | | AU 5403294 A | 26-04-1994 |
| | | CA 2145983 A1 | 14-04-1994 |
| | | DE 69309479 D1 | 07-05-1997 |
| | | EP 0662843 A1 | 19-07-1995 |
| | | JP 8502075 T | 05-03-1996 |
| | | WO 9407542 A2 | 14-04-1994 |
| | | AT 205728 T | 15-10-2001 |
| | | AU 686119 B2 | 05-02-1998 |
| | | AU 5353194 A | 26-04-1994 |
| | | CA 2146239 A1 | 14-04-1994 |
| | | DE 69330794 D1 | 25-10-2001 |
| | | DE 69330794 T2 | 23-05-2002 |
| | | DK 670736 T3 | 05-11-2001 |
| | | EP 0670736 A1 | 13-09-1995 |
| | | ES 2168098 T3 | 01-06-2002 |
| | | JP 8511765 T | 10-12-1996 |
| | | PT 670736 T | 28-03-2002 |
| | | WO 9407535 A2 | 14-04-1994 |
| US 5346686 A | 13-09-1994 | AT 205728 T | 15-10-2001 |
| | | AU 686119 B2 | 05-02-1998 |
| | | AU 5353194 A | 26-04-1994 |
| | | CA 2146239 A1 | 14-04-1994 |
| | | DE 69330794 D1 | 25-10-2001 |
| | | DE 69330794 T2 | 23-05-2002 |
| | | DK 670736 T3 | 05-11-2001 |
| | | EP 0670736 A1 | 13-09-1995 |
| | | ES 2168098 T3 | 01-06-2002 |
| | | JP 8511765 T | 10-12-1996 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 03 00 0204

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2003

| Patent document cited in search report | | Publication date | Patent family member(s) | | | Publication date |
|---|---|---|---|---|---|---|
| US 5346686 | A | | PT | 670736 | T | 28-03-2002 |
| | | | WO | 9407535 | A2 | 14-04-1994 |
| | | | US | 5605671 | A | 25-02-1997 |
| WO 9323085 | A | 25-11-1993 | AT | 196094 | T | 15-09-2000 |
| | | | AU | 677208 | B2 | 17-04-1997 |
| | | | AU | 4384593 | A | 13-12-1993 |
| | | | CA | 2136330 | A1 | 25-11-1993 |
| | | | DE | 69329382 | D1 | 12-10-2000 |
| | | | DE | 69329382 | T2 | 15-03-2001 |
| | | | DK | 641222 | T3 | 11-12-2000 |
| | | | EP | 0641222 | A1 | 08-03-1995 |
| | | | EP | 1004322 | A2 | 31-05-2000 |
| | | | ES | 2150945 | T3 | 16-12-2000 |
| | | | JP | 3380738 | B2 | 24-02-2003 |
| | | | JP | 10291939 | A | 04-11-1998 |
| | | | JP | 2941057 | B2 | 25-08-1999 |
| | | | JP | 7508289 | T | 14-09-1995 |
| | | | WO | 9323085 | A1 | 25-11-1993 |
| | | | US | 6083481 | A | 04-07-2000 |
| | | | US | 5849260 | A | 15-12-1998 |
| | | | US | 5925331 | A | 20-07-1999 |
| | | | US | 5879658 | A | 09-03-1999 |
| | | | US | 5888474 | A | 30-03-1999 |
| | | | US | 5968476 | A | 19-10-1999 |
| | | | US | 6074627 | A | 13-06-2000 |
| | | | US | 6248304 | B1 | 19-06-2001 |
| WO 0020594 | A | 13-04-2000 | AU | 6278399 | A | 26-04-2000 |
| | | | WO | 0020594 | A1 | 13-04-2000 |



For more details about this annex : see Official Journal of the European Patent Office, No. 12/82